# EUROPEAN PATENT SPECIFICATION

(11) **EP 2 352 025 B1**
(45) Date of publication and mention of the grant of the patent: **15.07.2020**
(21) Application number: 09806724.2
(22) Date of filing: 11.08.2009
(51) Int. Cl.: G01N 33/49, G01N 33/86, B01L 3/00

(54) **BLOOD-PLATELET TEST METHOD**
THROMBOZYTENTESTVERFAHREN
MÉTHODE DE TEST DE PLAQUETTES SANGUINES

(30) Priority: 11.08.2008 JP 2008207389; 25.12.2008 JP 2008330222
(43) Date of publication of application: 03.08.2011
(73) Proprietor: FUJIMORI KOGYO CO., LTD., Tokyo 160-0023 (JP)
(72) Inventor: HOSOKAWA, Kazuya, Tokyo 160-0023 (JP); FUKASAWA, Masashi, Tokyo 160-0023 (JP); GONDA, Maki, Tokyo 160-0023 (JP)
(74) Representative: Henderson, Helen Lee
(86) International application number: PCT/JP2009/064202
(87) International publication number: WO 2010/018833

(56) References cited:
- EP-A1- 1 950 567
- WO-A1-98/00231
- WO-A1-2006/115844
- WO-A1-2009/069656
- JP-A- 2002 277 479
- JP-A- 2004 004 002
- JP-A- 2006 145 345
- JP-A- 2006 145 345
- JP-A- 2007 271 323
- JP-T- 11 506 215
- JP-T- 2002 528 703
- JP-T- 2006 501 449
- US-A1- 2007 254 325

## Description

### TECHNICAL FIELD

The present invention relates to a method for measuring platelet function of blood using a small amount of the blood, more particularly, to a method using a microchip for testing platelet function of blood.

### BACKGROUND ART

### (Problems in Prior Art of Platelet Aggregation Test)

Activation and aggregation of platelets play a central role in thrombus formation (white thrombus) in the artery and primary hemostasis.

Platelets directly and indirectly bind to collagen existing under the vascular endothelial cells when a blood vessel is damaged. Under an environment with slow blood flow (under low shear stress), their binding is mainly direct binding via collagen receptors such as GPVI, while under an environment with fast blood flow (under high shear stress), vWF binds to collagen and the GPIbα receptor of platelets binds to vWF, causing indirect binding of the platelets to collagen. Direct and indirect interactions with collagen activate platelets, and by such stimulation, various platelet-activating substances such as ADP and serotonin are released from dense granules and α-granules.

Those released platelet-activating factors activate their own platelets and platelets in their vicinity. In activated platelets, fibrinogen receptors GPIIb and IIIa are structurally altered to the activated forms, changing the platelets to the high affinity form against fibrinogen. Through fibrinogen which is dimeric, activated platelets are sequentially cross-linked to each other, leading to platelet aggregation.

However, most of the conventional aggregometers employs a method to measure activation and the aggregation process of platelets caused by stimulation with a large amount of a platelet-activating reagent (Non-patent Document 1).

Therefore, the platelet activation reaction is induced under an environment which is very different from the physiological platelet activation condition, and, although it has been possible to measure an evident difference in the function such as congenital dysfunction of each receptor, it has been difficult to measure more physiological platelet functions.

PFA-100 (Platelet Function Analyzer: Non-patent Document 2) is a system to measure obstruction of holes by the overall activation of platelets due to contact with immobilized collagen, shear stress and contact with a platelet-inducing substance. Compared to conventional platelet aggregation by stimulation with a single platelet-activation induction substance, this is a measurement system employing an environment more similar to the physiological environment. However, it has been impossible to control variation of data and the concentrations of induction substances contained in the blood passing through the holes. Therefore, platelet aggregation induced under a condition in which platelet-activation induction substances are at very low concentrations or absent, which is observed in patients where platelets are already activated by development of thrombosis; and platelet aggregability caused by stimulation with a very high concentration of a platelet-inducing substance in the case of measurement of platelet function in patients suffering from platelet dysfunction; could not be appropriately measured.

Further, Patent Document 1 discloses a method for measuring platelet function where blood is allowed to pass through the inside of a capillary and then through the opening of a partitioning member, and the length of time required for obstruction of the opening of the partitioning member by thrombus formation is measured. However, since, in this method, a large amount of a platelet-activating reagent is added, it has been difficult to measure platelet function in detail reflecting *in vivo* conditions in cases, for example, where platelets are activated but the number of platelets is reduced and where the number of platelets is normal but platelet function is weak.
Patent document 2 describes a method for measuring platelet function, which utilizes arachidonic acid at a sufficient concentration to maximally activate platelets. As with Patent Document 1, it is difficult to measure platelet function with sufficient accuracy to reflect *in vivo* conditions, particularly in patients where platelets are activated but the number of platelets is reduced or where the number of platelets is normal but platelet function is weak.
Patent document 3 describes an apparatus and method for monitoring thrombus formation in which anticoagulated blood is flowed through a channel simulating a blood vessel while releasing an anticoagulant treatment or promoting blood coagulation in order to monitor thrombus formation. However, Patent Document 2 fails to teach a method for monitoring platelet aggregation alone.

### PRIOR ART DOCUMENTS

### Patent Document

Patent Document 1: JP 2007-298511 A/US 2007/0254325
Patent Document 2 : WO 2006/115844
Patent Document 3 : EP 1950567

### Non-patent Documents

Non-patent Document 1: "Platelet Aggregability Test", Thrombosis and Circulation, vol. 12, No.4, p 17-20, 2004
Non-patent Document 2: "Measurement of Platelet Aggregability with PFA-100" Thrombosis and Circulation, vol. 13, No. 3, p90-94, 2005

### SUMMARY OF INVENTION

In diseases such as sepsis and disseminated intravascular coagulation syndrome (DIC), platelets are in an activated state due to vascular endothelial disorder and thrombus formation, and complexes between platelets and leukocytes and the like are also formed. Further, since platelets are extremely consumed by continuous thrombus formation, a bleeding episode may occur in spite of thrombus formation *in vivo.*

In conventional platelet function tests, it has been difficult to carry out an assay which strictly reflects such symptoms.

For example, in the turbidimetric method, when the reactivity against a platelet-inducing substance is promoted, platelet function is judged to be promoted (strong) even in cases where platelets are reduced. Further, since the complexes between platelets and leukocytes, which are formed by inflammatory response or the like *in vivo* and largely affect thrombus formation, precipitate together with erythrocytes during centrifugation for preparation of platelet-rich plasma, they are not included in platelet-rich plasma.

Further, when PFA-100 is used, the same concentration of an induction substance is employed for the measurement both in patients having strong platelet function and in patients having weak platelet function, so that it is impossible to appropriately carry out a test to confirm natural aggregation induction under a condition where the induction substance is absent or at a very low concentration or to confirm, in a patient to whom an anti-platelet agent was administered, the pharmacological effect of the agent under a condition where the concentration of the induction substance is very high. Further, even in cases where the obstruction time delayed in measurement with PFA-100, it has been difficult to carry out comparison between the cases where platelet function is weak and where the number of platelets is small but the platelet function is promoted (activated in the living body).

The present invention was made in view of the above-described circumstances and aims to provide a method which enables efficient and accurate evaluation of platelet function under an environment equivalent to the blood flow, using a small amount of blood.

To solve the problems, the present invention provides a method for testing platelet aggregation, wherein platelet aggregation is tested by subjecting anticoagulated blood to a weak platelet-activation treatment with a platelet-activating reagent, passing the treated anticoagulated blood through a capillary having a platelet-adhesive surface on at least a part of its inner surface, and observing or measuring the behavior of the blood in the capillary to measure platelet aggregation, wherein at least a part of the inside of said capillary has a section comprising walls which extend along the direction of the blood flow in the capillary and divide the width of the capillary into not less than 5 channels, wherein the width of each channel in said section is 10 to 200 µm,
wherein said platelet-adhesive surface is provided in said section of the capillary, and
wherein said weak platelet-activation treatment is carried out by mixing the anticoagulated blood with a platelet-activating reagent in an amount with which irreversible platelet aggregation does not occur.

Here, the anticoagulated blood is preferably a whole blood treated with an anticoagulant selected from citric acid, heparin or hirudin.

Further, the platelet-adhesive surface is preferably made of collagen coating or glass.

Here, the platelet-activation treatment is preferably carried out with a platelet-activating reagent, and the platelet-activating reagent is preferably adenosine diphosphate, which is mixed with the anticoagulated blood to a concentration of 0.001 to 5 µM, or the platelet-activating reagent is preferably arachidonic acid, which is mixed with the anticoagulated blood to a concentration of 0.001 to 1 mM.

The capillary is preferably formed in a microchip.

In the method of the present invention for testing platelet aggregation, platelet aggregation is preferably tested by a process wherein the anticoagulated blood, subjected to weak platelet-activation treatment, is introduced into the capillary by a pump, and the pressure exerted on the pump by inflow of the blood into the capillary is measured with a pressure sensor. Here, the anticoagulated blood is preferably stored in a blood storage section connected to the capillary and the pump, which blood is introduced into the capillary by introduction of a liquid having a specific gravity smaller than the blood into the blood storage section by the pump, and the inflow pressure of the liquid is measured, thereby indirectly measuring the pressure exerted by inflow of the blood into the capillary.

Further, the anticoagulated blood is preferably stored in a blood storage section connected to the capillary and the pump, which anticoagulated blood is mixed with the platelet-activating reagent in the blood storage section by introduction of the platelet-activating reagent by the pump into the blood storage section, and the blood mixed with the platelet-activating reagent is introduced into the capillary, while measuring the inflow pressure of the platelet-activating reagent, thereby indirectly measuring the pressure exerted by inflow of the blood into the capillary.

Further, preferably, in the method of the present invention for testing platelet aggregation, the capillary is connected to the blood storage section, and the anticoagulated blood mixed with the platelet-activating reagent in the blood storage section is allowed to pass through the capillary. Here, the platelet-activating reagent is preferably mixed with the anticoagulated blood immediately before the measurement. Alternatively, the platelet-activating reagent is mixed with the anticoagulated blood such that the concentration of the platelet-activating reagent in the mixture of the anticoagulated blood and the platelet-activating reagent increases along a linear concentration gradient or a stepwise concentration gradient. The mixture of the anticoagulated blood and the platelet-activating reagent is preferably stirred.

### EFFECTS OF THE INVENTION

With the method for testing platelet aggregation according to claim 1 of the present invention, platelet aggregation can be tested by allowing anticoagulated blood to pass through a capillary having a platelet-adhesive surface on at least a part of its inner surface, and observing or measuring the behavior of the blood in the capillary, using as an index activation of platelets by the shear stress caused when the blood passes through the platelet-adhesive surface.

With the method for testing platelet aggregation according to claim 2 of the present invention, the anticoagulation treatment can be carried out inexpensively since citric acid, heparin or hirudin is used for the anticoagulation treatment.

In cases where collagen coating is used as the platelet-adhesive surface and the anticoagulant to be used is a calcium chelator such as citric acid, measurement of platelet function is carried out more suitably by mixing blood with a platelet-activating reagent and allowing the mixture to pass through the collagen coating. On the other hand, in cases where an anticoagulant other than a calcium chelator, especially hirudin, is used, platelet aggregation occurs more rapidly and strongly on collagen than in cases where the anticoagulation treatment is carried out with citric acid, causing obstruction of the capillary, so that a stable platelet function test is possible even without using a platelet-activating reagent. When platelet aggregation does not occur because of platelet dysfunction, use of an anti-platelet agent, or the like, the extent of decrease in platelet function or the effect of the anti-platelet agent can be measured by mixing of a platelet-activating reagent before the measurement. By arranging channels having different channel widths in a single chip and using bloods anticoagulated with hirudin and citric acid, the strength of platelet aggregability, the sensitivity to a platelet-inducing substance, the effect of an anti-platelet agent, and the like can be comprehensively measured.

With the method for testing platelet aggregation according to claim 3 of the present invention, more physiological platelet function can be measured since the platelet-adhesive surface is made of collagen coating.

With the method for testing platelet aggregation according to claim 3 of the present invention, platelet function can be measured with a substance which is less expensive, since the platelet-adhesive surface is made of glass.

With the method for testing platelet aggregation according to claim 1 of the present invention, platelet function can be measured under a more physiological condition and test results reflecting various diseases can be obtained, since the anticoagulated blood is subjected to weak platelet-activation treatment.

With the method for testing platelet aggregation according to claim 1 of the present invention, weak platelet-activation treatment can be simply carried out since the weak platelet-activation treatment is carried out by mixing the anticoagulated blood with a platelet-activating reagent in an amount with which irreversible platelet aggregation does not occur, and a combination of platelet activation by a platelet-activating reagent and platelet activation by shear stress on the platelet-adhesive surface allows observation of more physiological platelet activation.

With the method for testing platelet aggregation according to claim 4 of the present invention, platelet function can be tested under more physiological conditions and results reflecting various diseases can be obtained since the platelet-activating reagent is adenosine diphosphate which is mixed with the anticoagulated blood to a concentration of 0.001 to 5 µM.

With the method for testing platelet aggregation according to claim 4 of the present invention, platelet function can be tested under more physiological conditions and results reflecting various diseases can be obtained since the platelet-activating reagent is arachidonic acid which is mixed with the anticoagulated blood to a concentration of 0.001 to 1 mM.

With the method for testing platelet aggregation according to claim 1 of the present invention, platelet activation by shear stress easily occurs since at least a part of the inside of the capillary has a ing section comprising walls which extend along the direction of the blood flow in the capillary and divide the width of the capillary into not less than 5 channels.

With the method for testing platelet aggregation according to claim 1 of the present invention, small platelet aggregates, when formed, can increase the internal pressure without being swept away by the blood flow even with fast blood flow and under high shear stress since the width of each channel in the section is 10 to 200 µm, which allows the channel to provide a scaffold.

With the method for testing platelet aggregation according to claim 1 of the present invention, platelet aggregates are easily retained in the section since the platelet-adhesive surface is provided in the section of the capillary.

With the method for testing platelet aggregation according to claim 5 of the present invention, a test can be carried out with a small amount of blood since the capillary is formed in a microchip.

With the method for testing platelet aggregation according to claim 6 of the present invention, platelet function can be quantitatively measured since the anticoagulated blood is introduced into the capillary by a pump, and the pressure exerted on the pump by inflow of the blood into the capillary is measured with a pressure sensor, thereby testing platelet function.

With the method for testing platelet aggregation according to claim 7 of the present invention, the pump is not contaminated with blood since the anticoagulated blood is stored in a blood storage section connected to the capillary and the pump, which blood is introduced into the capillary by introduction of a liquid having a specific gravity smaller than the blood into the blood storage section by the pump, and the inflow pressure of the liquid is measured, thereby indirectly measuring the pressure exerted by inflow of the blood into the capillary.

With the method for testing platelet aggregation according to claim 8 of the present invention, a test can be quickly carried out since the anticoagulated blood is stored in a blood storage section connected to the capillary and the pump, which anticoagulated blood is mixed with a platelet-activating reagent in the blood storage section by introduction of the platelet-activating reagent by the pump into the blood storage section, and the blood mixed with the platelet-activating reagent is introduced into the capillary, while measuring the inflow pressure of the platelet-activating reagent, thereby indirectly measuring the pressure exerted by inflow of the blood into the capillary.

With the method for testing platelet aggregation according to claim 9 of the present invention, platelet activation by the platelet-activating reagent in a wide range of concentration can be measured and hyperactivity and hypoactivity of platelets can be measured in a single experiment, which is desirable, since the platelet-activating reagent is mixed with the anticoagulated blood such that the concentration of the platelet-activating reagent in the mixture of the anticoagulated blood and the platelet-activating reagent increases along a linear concentration gradient or a stepwise concentration gradient.

With the method for testing platelet aggregation according to claim 10 of the present invention, more accurate test results can be obtained since the mixture of the anticoagulated blood and the platelet-activating reagent is stirred.

### BRIEF DESCRIPTION OF THE DRAWINGS

Fig. 1 is a diagram showing a first microchip constituting a platelet monitoring device not according to the invention that can be used in the method for testing platelet aggregation.
Fig. 2 is a diagram showing a first platelet monitoring device not according to the invention that can be used in the method for testing platelet aggregation.
Fig. 3 is a diagram showing a second microchip constituting a platelet monitoring device not according to the invention that can be used in the method for testing platelet aggregation.
Fig. 4 is a diagram showing a second platelet monitoring device not according to the invention that can be used in the method for testing platelet aggregation.
Fig. 5 is a diagram showing changes in the pressure in the pump 109 in Example 1.
Fig. 6 is a diagram showing changes in the pressure in the pump 109 in Example 2.
Fig. 7 is a diagram showing changes in the pressure in the pump 109 in Example 3.
Fig. 8 is a diagram showing changes in the pressure in the pump 109 in Example 4.
Fig. 9 is a diagram showing changes in the pressure in the pump 109 in Example 5.
Fig. 10 is a diagram showing changes in the pressure in the pump 109 in Example 6.
Fig. 11 is a diagram (photographs) showing the state of platelet activation in Example 7. A: no ADP; B: 0.025 µM ADP; C: 0.05 µM ADP; D: 0.1 µM ADP
Fig. 12 is a diagram showing changes in the pressure in the pump 209 in Examples 8 to 10.
Fig. 13 is a diagram showing a third platelet monitoring device not according to the invention that can be used in the method for testing platelet aggregation.
Fig. 14 is a diagram showing changes in the pressure in the pump 209 in Examples 11 to 13.
Fig. 15 is a diagram showing changes in the pressure in the pump 209 in Examples 14 and 15.
Fig. 16 is a diagram showing the results on pressure increase at 20 µl/min., which results were obtained using blood to which arachidonic acid was added. A shows the results on pressure increase at 20 µl/min., which results were obtained using the blood of the subject G in Example 16a to which arachidonic acid was added. B shows pressure increase in an experiment similar to 16a, wherein the subject G had preliminarily taken 100 mg of aspirin.
Fig. 17 is a diagram showing the results on pressure increase at 10 µl/min., which results were obtained using blood to which arachidonic acid was added. C shows the results on pressure increase at 10 µl/min., which results were obtained using the blood of the subject G in Example 16b to which arachidonic acid was added. D shows pressure increase in an experiment similar to 16b, wherein the subject G had preliminarily taken 100 mg of aspirin.
Fig. 18 is a diagram showing the results on pressure increase at 5 µl/min., which results were obtained using blood to which arachidonic acid was added. E shows the results on pressure increase at 5 µl/min., which results were obtained using the blood of the subject G in Example 16C to which arachidonic acid was added. F shows pressure increase in an experiment similar to 16c, wherein the subject G had preliminarily taken 100 mg of aspirin.

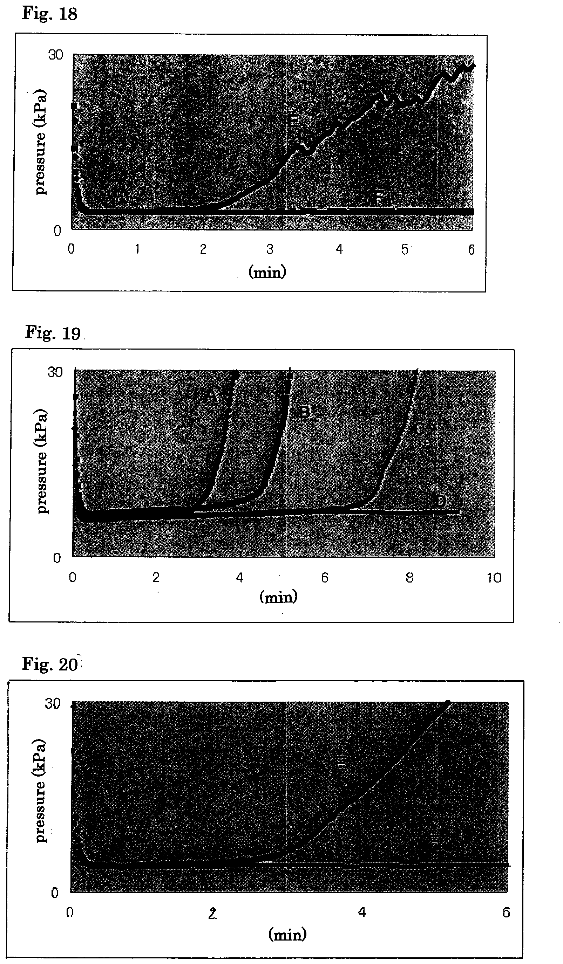

Fig. 19 is a diagram showing the results on pressure increase at 20 µl/min., which results were obtained using blood to which collagen was added. A shows the results on pressure increase at 20 µl/min., which results were obtained using the blood of the subject G in Example 16d to which 6 µl of collagen was added. B, C and D show pressure increase in experiments similar to Example 16d, wherein the blood to which 18 µl of collagen was added in Example 18 was used; wherein the blood to which 12 µl of collagen was added in Example 18 was used; and wherein the subject G had preliminarily taken 100 mg of aspirin; respectively.
Fig. 20 is a diagram showing the results on pressure increase at 10 µl/min., which results were obtained using blood to which collagen was added. E shows the results on pressure increase at 10 µl/min., which results were obtained using the blood of the subject G in Example 16e to which collagen was added. F shows pressure increase in an experiment similar to 16e, wherein the subject G had preliminarily taken 100 mg of aspirin.
Fig. 21 is a diagram showing the results on pressure increase at 5 µl/min., which results were obtained using blood to which collagen was added. F shows the results on pressure increase at 5 µl/min., which results were obtained using the blood of the subject G in Example 16f to which collagen was added. G shows pressure increase in an experiment similar to 16f, wherein the subject G had preliminarily taken 100 mg of aspirin.
Fig. 22 is a diagram showing the results on pressure increase at 20 µl/min., which results were obtained using the blood treated with 0.4 µg/ml or 0.8 µg/ml ReoPro in Example 21. The 'control' indicates the results on pressure increase at 20 µl/min., which results were obtained using the blood in Example 19 (without treatment with ReoPro).
Fig. 23 is a diagram showing the results on pressure increase at 7 µl/min., which results were obtained using the blood treated with 0.4 µg/ml or 0.8 µg/ml ReoPro in Example 21. The 'control' indicates the results on pressure increase at 7 µl/min., which results were obtained using the blood in Example 20 (without treatment with ReoPro).
Fig. 24 is a diagram showing the results on pressure increase at 20 µl/min., which results were obtained using the blood treated with 0.01 µg/ml or 0.1 µg/ml OS-1 in Example 22. The 'control' indicates the results on pressure increase at 20 µl/min., which results were obtained using blood without treatment with OS-1.
Fig. 25 is a diagram showing the results on pressure increase at 7 µl/min., which results were obtained using the blood treated with 0.01 µg/ml or 0.1 µg/ml OS-1 in Example 22. The 'control' shows the results on pressure increase at 7 µl/min., which results were obtained using blood without treatment with OS-1.

### MODES FOR CARRYING OUT THE INVENTION

A platelet testing device that can be used in the method is described referring to Figures. In the present disclosure, 'blood' includes whole blood and platelet-rich plasma.

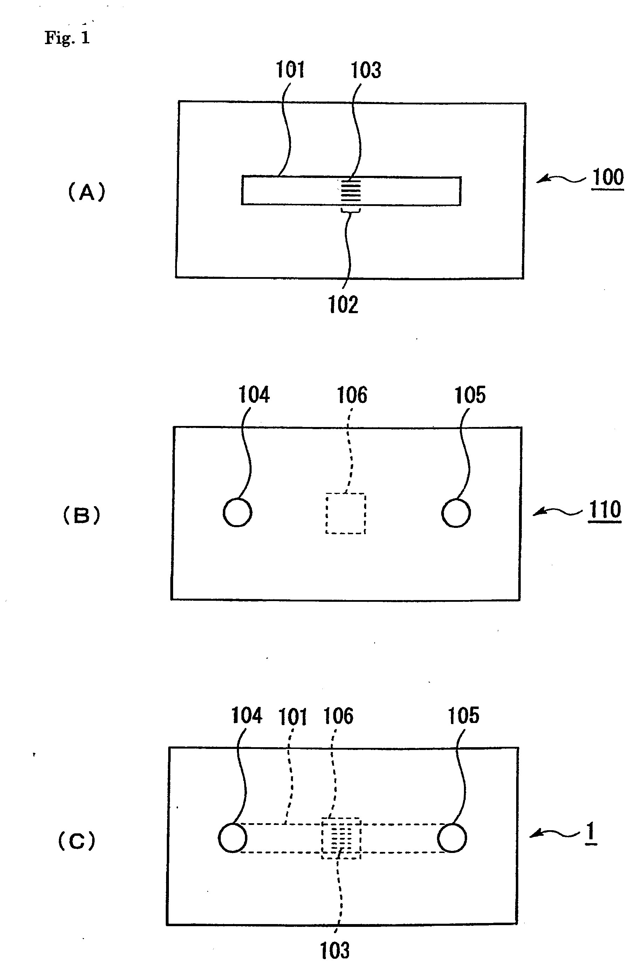

Fig. 1 is a conceptual diagram showing the first microchip constituting the platelet testing device not according to the invention that can be used in the method for testing platelet aggregation. Explanation will now be made based on Fig. 1.

Fig. 1(A) is a plan view showing the first substrate 100 wherein a groove corresponding to the capillary 101 of the microchip 1 is engraved on the surface. The cross-sectional shape of this groove is arbitrary and may be rectangular, U-shaped, V-shaped or the like. Since platelet aggregates are fragile, the depth of the groove is preferably not more than 10 to 200 µm for measuring pressure increase. The width of the groove is preferably 10 to 100 µm.

In a part between the first end (the end in the inlet side) and the second end (the end in the outlet side) of the groove corresponding to the capillary 101, a plurality of channel dividing walls 103 extending along the direction of the blood flow are provided to form the channel dividing section 102 which divides the width of the capillary into a plurality of channels.

Further, the intervals between the channel dividing walls 103 are preferably not more than 200 µm. With a width of not more than 200 µm, when a platelet aggregate was formed, the platelet aggregate can increase the internal pressure without being swept away by the blood flow even with fast blood flow and under high shear stress. Further, in the channel dividing section 102, the width of the capillary 101 is preferably divided into not less than 5 channels by the channel dividing walls 103. That is, in case where the width of the channel is divided into not less than 5 channels, obstruction of the respective divided channels are averaged, and therefore data with less variation can be easily obtained.

The shape of the channel dividing walls 103 is not restricted as long as they can divide the width of the capillary 101 into a plurality of channels.

Fig. 1(B) is a plan view showing the second substrate 110 on which penetrating holes corresponding to the inlet 104 and the outlet 105 of the microchip 1 are engraved. The positions of the penetrating holes corresponding to the inlet 104 and the outlet 105 are the positions corresponding, upon lamination with the first substrate 100, to the first end of the capillary 101 and the second end of the capillary 101, respectively, on the first substrate 100. Further, by coating, with collagen or the like, the back side of the second substrate 110 which covers the channel dividing section 102 on the first substrate 100, a platelet-adhesive surface 106 is formed. More particularly, as shown in Fig. 1(C), the collagen or the like is widely applied on the area to be used as the platelet-adhesive surface 106 in consideration of the safety margin.

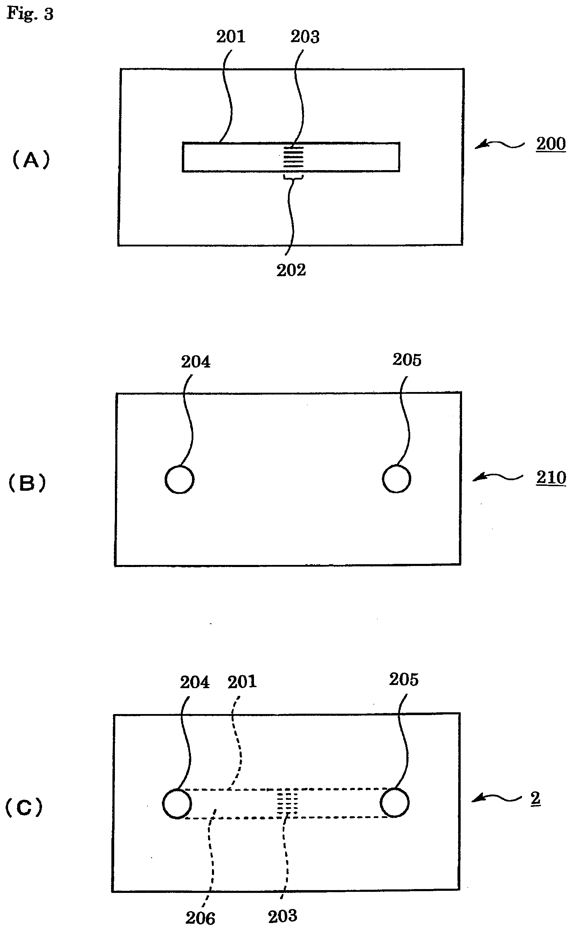

The platelet-adhesive surface may also extend over the entire length of the capillary as shown in Fig. 3 (second microchip). In the second microchip, the material of the second substrate 210 is glass, and the entire length of the second substrate side in the capillary 201 in the microchip 2 works as the platelet-adhesive surface 206.

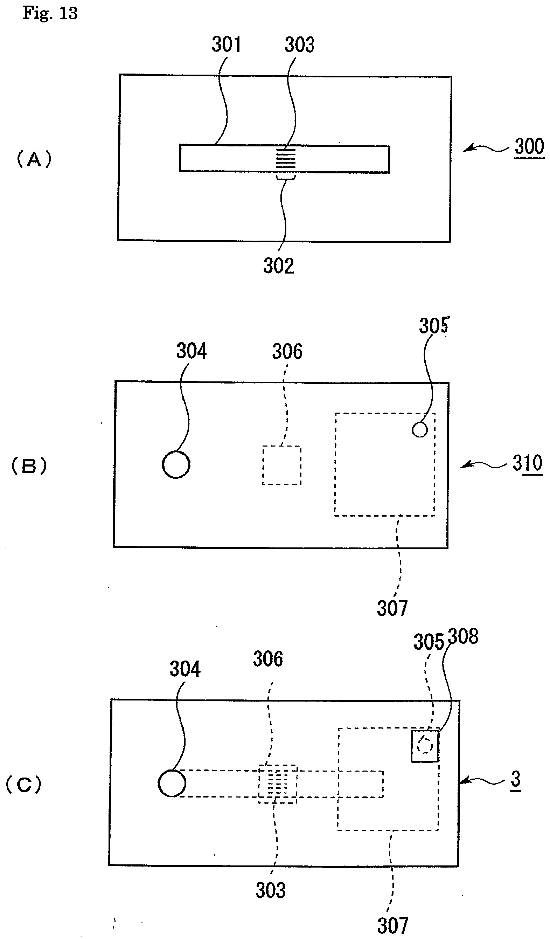

Alternatively, instead of providing a penetrating hole at the position corresponding to the second end of the capillary in the second substrate and thereby providing an outlet as shown in Figs. 1 and 3, a waste liquid reserving section 307 for storing blood waste which has passed through the platelet-adhesive surface 306 may be provided by providing a groove such that it surrounds the position corresponding to the second end of the capillary 301 on the second substrate 310 and covering the groove with the first substrate 300 as shown in Fig. 13. By setting the capacity of the waste liquid reserving section 307 larger than the amount of the blood to be tested, the operation of aspirating blood waste from the outlet with a pump or the like and discharging it as in the first and second microchips can be eliminated, so that the test can be carried out more easily. A penetrating hole may be provided in the waste liquid reserving section 307 to provide an air hole 305. By placing a blood absorbing material 308 at the position corresponding to the penetrating hole 305 on the surface of the microchip 3, dispersion of blood waste to the outside of the microchip can be avoided even in cases where the amount of the blood sample is large. Examples of the blood absorbing material to be attached include sponges and cloths.

Fig. 1(C) is a plan view showing the microchip 1 wherein the first substrate 100 and the second substrate 110 are laminated with each other such that the groove on the first substrate 100 and the platelet-adhesive surface of the second substrate 110 are facing inward. The wavy line indicates that the capillary 103 exists in the microchip 1.

Examples of the platelet-adhesive surface include collagen coating and glass. Among these, collagen is especially preferred since it can be easily obtained and handled, and can provide a model similar to an actual blood vessel. The platelet-adhesive surface may also be one containing collagen and tissue thromboplastin. The substance such as collagen is coated on the platelet-adhesive surface 106 with high adhesive strength to avoid being washed away by blood flow.

The collagen coating can be simply applied with high adhesive strength by, for example, dissolving collagen into an acidic solution and applying the resulting solution to a predetermined position on a substrate such as glass or polystyrene to which hydrophilicity was given, followed by washing and drying the substrate, as described in JP 05-260950 A and Blood. 1995 Apr 1;85(7):1826-35.

In cases where a hydrophobic resin or the like is to be coated, the coating can be achieved by hydrophilizing the surface of the resin by plasma treatment or the like and then applying a collagen solution to the desired area, followed by air drying or drying under reduced pressure.

In cases where a plastic is used as the base material, it can be easily coated with collagen or collagen containing tissue thromboplastin by hydrophilizing its surface by plasma treatment or the like and then applying a collagen solution to the desired area using a dispenser such as a pipette or a syringe.

In cases where glass is used as the platelet-adhesive surface, a second substrate 110 wherein only the position corresponding to the platelet-adhesive surface 106 is formed with glass and the other area is formed with a plastic, silicone or the like may be used, or, as shown in Fig. 3, the platelet-adhesive surface 206 may extend over the entire length of the capillary 201 in cases where a glass substrate is used as the second substrate 210 and the second substrate is laminated with a first substrate 200 formed with a plastic, silicone or the like.

The material of the microchip 1 is preferably a metal, glass, plastic, silicone or the like. In view of usage in blood monitoring (image analysis, especially), a transparent material is preferred. Further, in view of formation of a circuit, a plastic is preferred, and a transparent plastic is especially preferred. In cases where the material is a silicone such as PDMS (polydimethylsiloxane), excellent adhesion is attained between the substrates, so that the first substrate 100 can be laminated with the second substrate 110 even by pressing without using an adhesive or the like for adhesion, but in cases where a high pressure is applied to the inside of the microchip 1 , an adhesive is preferably used. It is also possible to easily and effectively suppress blood coagulation at unexpected sites by using poly(2-methoxyethyl acrylate) (PMEA). The groove and the hole provided on the substrate of the microchip 1 can be engraved with a cutter or laser beam, and in cases where the material of the microchip 1 is a plastic, they can also be formed by injection molding. Formation by injection molding is preferred since microchips 1 having a constant quality can be prepared efficiently.

An example of platelet function test using the microchip 1 will now be described based on Fig. 1(C). To a first inlet 104, a tube which is not shown is connected, and through the tube, a blood reservoir (blood storage section) 107 and a liquid sending pump 109, which are not shown, are connected. By injecting the liquid in the connected pump 109 into the reservoir 107, blood in the reservoir is injected to the microchip 1. If the liquid in the pump is a platelet-activating reagent, platelets of the anticoagulated blood in the reservoir are activated, and they are further activated due to shear stress when they pass through the platelet-adhesive surface. Further, the concentration of the platelet-activating reagent in the reservoir may be continuously increased. By placing a stirring bar in the blood reservoir and stirring the blood therein, the platelet-activating reagent is constantly mixed with the blood, which is preferred.

The liquid in the liquid sending pump may be a liquid having a specific gravity smaller than the blood, such as mineral oil or physiological saline, and the liquid may be introduced into the reservoir preliminarily filled with anticoagulated blood, thereby allowing the blood to be introduced into the capillary. By measuring the inflow pressure of the liquid, the pressure exerted by inflow of the blood into the capillary can be indirectly measured. The anticoagulated blood with which the reservoir is preliminarily filled is preferably a mixture with a platelet-activating reagent. It is also possible to place a platelet-activating reagent in the reservoir in advance in the dry or liquid state, followed by mixing it with the blood. Further, by increasing the flow rate of the liquid sending pump in a stepwise manner, it is also possible to increase the shear stress in a stepwise manner.

Examples of the anticoagulant used for anticoagulation treatment to suppress blood coagulation include sodium or potassium citrate; sodium or potassium oxalate; ACD (Acid Citrate Dextrose); and ethylenediaminetetraacetic acid (EDTA) salts. Such an anticoagulant may be used as a powder, freeze-dried product or solution such as an aqueous solution. Among these anticoagulants, 3.2% sodium citrate, which is commonly used, is preferred since it is easily available. In this case, it is preferred to use 1 volume of this anticoagulant for 9 volumes of blood.

Examples of the other anticoagulants which may be used include heparin, hirudin, thrombin aptamers and corn-derived trypsin inhibitor (1977. J. Biol. Chem 252. 8105). More than one anticoagulant may be used. In cases where the anticoagulant employed is hirudin, stronger platelet aggregation, compared to the case of anticoagulation treatment with citric acid, occurs even without treatment with a platelet-activating reagent, so that hirudin is suitable for measurement of shear stress-dependent platelet function. In the case of blood anticoagulated with citric acid, measurement of platelet function dependent on stimulation by a platelet-activating reagent can be accurately carried out, so that citric acid is more suitable for evaluation of an anti-platelet agent and the like.

Examples of the method to obtain the anticoagulated blood include a method wherein the above-described anticoagulant is preliminarily placed in a syringe or a vacuum blood collection tube, followed by collecting blood therewith, and a method wherein the anticoagulant is quickly added to blood immediately after collection of the blood.

It is also possible to collect blood with a vacuum blood collection tube or the like containing heparin, followed by adding heparinase and an anticoagulant suitable for the monitoring purpose, thereby degrading the heparin with the heparinase, to achieve replacement of the heparin with the anticoagulant suitable for the measuring purpose.

Examples of the platelet-activating reagent to be mixed with the anticoagulated blood include ADP, collagen, thrombin, arachidonic acid and ristocetin. The platelet-activating reagent is mixed with the anticoagulated blood at a concentration at which weak platelet activation occurs. The concentration at which weak platelet activation occurs is a concentration at which irreversible platelet aggregation (secondary aggregation of platelets) does not occur under a static state. For example, in the case of ADP, it is 0.001 to 5 µM, and in the case of arachidonic acid, it is 0.001 to 1 mM. However, in cases where the reactivities to these platelet-activating reagents are clearly decreased due to abnormality of platelet function or administration of an anti-platelet agent, measurement of the extent of the decrease is preferably carried out with addition of a platelet-activating reagent to a concentration higher than these concentrations. In particular, in cases where the extent of the effect of clopidogrel or aspirin is measured, it is preferred to carry out the measurement with an increased concentration of ADP or arachidonic acid, respectively.

The mixture of the weak platelet-activating reagent and the anticoagulated blood which has been introduced from the inlet 104 and allowed to pass through the capillary 101 passes through the channel dividing section 102 while causing shear stress and thereby enhancing the activation of platelets, leading to adhesion and accumulation of the platelets on the platelet-adhesive surface. By adjusting the flow rate and the microchip and carrying out comparative experiments under high shear stress and low shear stress, more detailed evaluation of platelet function, evaluation of the pharmacological effect of an anti-platelet agent, and the like are possible. By observing the flow rate and the property of the mixture passing through the channel dividing section 102, platelet function can be monitored. The blood subjected to the monitoring is discharged from the outlet 105 provided at the end of the capillary 101.

A platelet testing device using the microchip 1 will now be described.

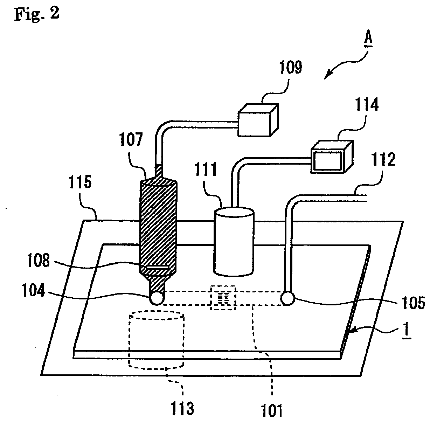

Fig. 2 is a conceptual diagram of a platelet testing device A, wherein the microchip 1 is constructed with transparent substrates and incorporated into the device. The first device will now be described based on Fig. 2.

To an inlet 104 of the microchip 1, a reservoir 107 (blood storage section), wherein anticoagulated blood is placed and a stirring bar 108 is contained, is connected in an inverted position, and to the reservoir 107, a liquid sending pump 109 for supplying a platelet-activating reagent is connected via a tube. To the liquid sending pump 109, a pressure sensor which is not shown is connected.

From the liquid sending pump 109, the platelet-activating reagent is injected into the reservoir 107, and the platelet-activating reagent and the anticoagulated blood are mixed together by the stirring bar 108 which is driven by a magnetic stirrer 113.

By adjusting the flow rate of the pump when the platelet-activating reagent is introduced from the liquid sending pump 109 into the reservoir 107, and thereby making the concentration of the platelet-activating reagent in the mixture with blood automatically increase from 0 to a concentration at which platelets can be sufficiently induced, promotion of function of platelets and decrease in function of platelets can be measured in a single experiment, which is preferred. For example, the ADP concentration may be increased along a concentration gradient from 0 to 0.1 µM, or the concentration of the induction substance may be increased from 0 to 0.02, 0.04, 0.06, 0.08 and 0.1 µM in a stepwise manner.

The anticoagulated blood mixed with the platelet-activating reagent is injected into the capillary 101 of the microchip 1. The mixture passes through the capillary 101 and reaches the channel dividing section 102 having a platelet-adhesive surface such as collagen or glass.

By observing activation of platelets (adhesion, aggregation and the like) in the channel dividing section 102 and obstruction of the capillary caused thereby using a camera 111, platelet function can be tested. Alternatively, a more quantitative platelet function test is possible by measuring the pressure in the channel with the pressure sensor connected to the liquid sending pump 109. Further, also by measuring the length of time required for the mixture of the blood and the platelet-activating reagent to pass through the capillary 101 or the amount of the mixture which has passed through the capillary 101, a platelet function test can be carried out.

The camera 111 is connected to an image analyzer 114, and these enable imaging and the like of the state of platelet activation. The combination of visual evaluation by taking images of platelet activation inside the capillary and a quantitative test for platelet activation based on pressure increase is very important in view of overall judgment of the condition of the blood from a patient. For example, in cases where platelets are already activated *in vivo* but remarkably consumed and reduced in a diseased state such as DIC, the pressure increase and the obstruction are delayed. Even in such cases, taking images of the inside with the camera enables confirmation of increased adhesion and aggregation and the like of platelets on the platelet-adhesive surface from immediately after the beginning of the test, so that overall judgment of the condition of the blood from a patient is possible.

The camera 111 may be movable along the direction of the blood flow in the capillary 101.

Platelets may be analyzed also by fluorescently labeling platelets with quinacrine or the like. In such a case, the monitoring results may be digitized by monitoring the luminance per unit area due to fluorescence by image analysis, thereby obtaining the monitoring results as data.

The microchip 1 is placed on a stage-shaped heater 115, and by heating the microchip 1 to 37°C with the heater 115, the monitoring can be carried out under a condition similar to the inside of the body.

The mixture containing blood which has passed through the channel dividing section 102 is smoothly discharged from the outlet 105 to the outlet duct 112.

The stirring bar is preferably subjected to anticoagulation treatment. The anticoagulation treatment may be coating of the surface of a magnetic material with heparin, polyvinyl lactonamide (PVLA), poly(2-methoxyethyl acrylate) (PMEA) or the like by soaking the magnetic material in a molten resin or formation of the surface of a stirring bar therewith by resin finishing such as in-mold injection molding

Although the device, wherein a platelet-activating reagent and anticoagulated blood are mixed together in a reservoir outside the microchip, followed by being introduced into the capillary in the microchip, was explained as above based on an example, the platelet function testing device is not restricted to the above-described example.

For example, it is also possible to construct a device wherein a mixing section to mix anticoagulated blood and a platelet-activating reagent together is provided in a microchip and the blood and the platelet-activating reagent are mixed together in the microchip, after which the mixture is allowed to flow into the capillary from the mixing section.

### EXAMPLES

The present invention will now be described in more detail by way of specific Examples, but the present invention is not limited thereto.

### [Preparation of Microchip and Platelet Function Testing Device]

Two transparent substrates, that is, a first substrate 100 shown in Fig. 1(A) and a second substrate 110 shown in Fig. 1(B) (injection-molded products manufactured by Richell Corporation) were prepared. The side of the first substrate 100 wherein the groove corresponding to a capillary 101 opens and the side of the second substrate 110 having a platelet-adhesive surface 106 were bonded together such that these face to each other using a silane coupling agent and thermocompression bonding at 60°C for 16 hours, to provide the microchip 1 shown in Fig. 1(C). In the first substrate 100, the length, depth and width of the channel (capillary) 101 were 20 mm, 50 µm and 2 mm, respectively. In terms of the channel dividing section 102, dividing walls 103 each having a length of 2 mm, width of 25 µm and height of 50 µm were placed at equal intervals of 25 µm, and this part was used as a channel dividing section 102. In the second substrate 100, each of the holes corresponding to an inlet 104 and an outlet 105 was a penetrating hole having a circular cross section and an inner diameter of 2 mm and a depth of 2 mm. At the position on the second substrate 110 which is overlapped with the channel dividing section 102 of the first substrate 100, 3 mg/ml collagen type I (manufactured by Nitta Gelatin Inc.) was applied and dried under vacuum to provide the platelet-adhesive surface 106.

As shown in Fig. 2, the prepared microchip 1 was placed on a stage-shaped heater 115, and a reservoir 107 was connected to the inlet 104 of the microchip 1. A pump109 was connected to the reservoir 107 via a tube, and the pressure exerted on the pump was measured with a pressure sensor which is not shown. In the reservoir 107, a cylindrical stirring bar 108 having a diameter of 2 mm and a length of 5 mm, which was prepared by coating an iron cylinder with PMEA, was contained. The stirring bar 108 was set such that it can be rotated at 60 to 180 rpm by the magnetic force of a stirrer 113 placed under the heater 115 which was heated to 37°C. An outlet duct 112 was connected to the outlet such that the blood after the analysis can be discharged. A camera 111 to which an image analyzer 114 was connected was placed above the channel dividing section 102 of the microchip 1, thereby allowing observation of the state of platelet activation in the channel dividing section 102.

### Example 1)

Using the microchip 1 and the platelet testing system A shown in Fig. 2, measurement was carried out. Blood subjected to anticoagulation treatment with citric acid was collected from the subject A.

The whole blood subjected to anticoagulation treatment with citric acid was filled in the reservoir 107, and physiological saline was injected from the inlet into the reservoir 107 by the pump 109 connected, thereby extruding the blood from the reservoir 107 and allowing the blood to flow into the microchip 1. In the reservoir 107, the stirring bar 108 is contained, and the blood is stirred by the stirring bar 108.

The flow rate of the pump 109 was 100 µl/min. for the first 10 seconds, and 3 µl/min. thereafter.

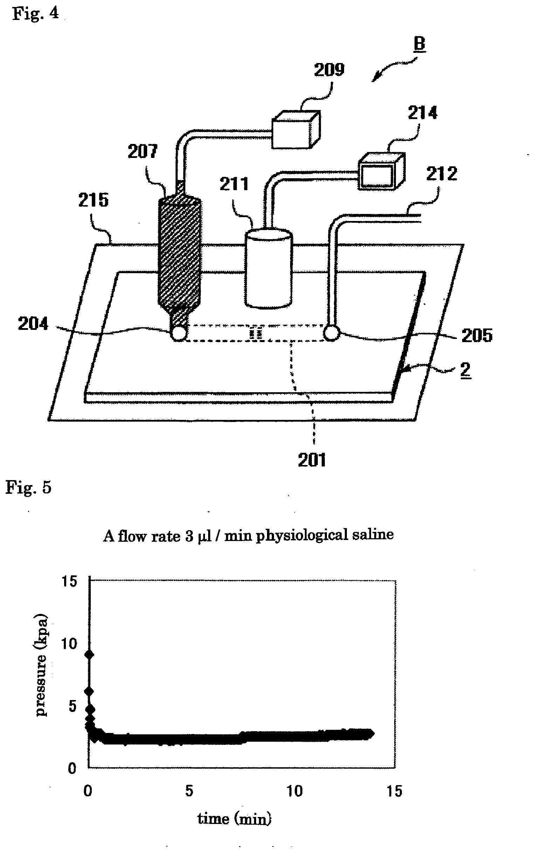

The changes in the pressure in the pump 109 are shown in Fig. 5.

### Example 2)

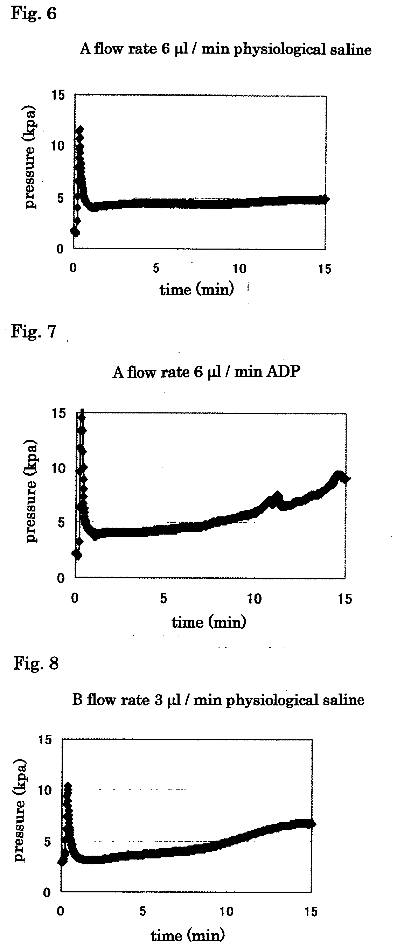

The experiment was carried out in the same manner as in Example 1 except that the flow rate was 6 µl/min. instead of 3 µl/min.
(Blood sample A from the same subject as in Example 1 was used.) The changes in the pressure in the pump109 are shown in Fig. 6.
(Discussion) By increasing the flow rate from 3 µl/min. to 6 µl/min. using the same sample, shear stress increased, and pressure increase was confirmed.

### Example 3)

The experiment was carried out in the same manner as in Example 2 except that 0.5 µM ADP solution was filled in the pump109 instead of physiological saline. (A blood sample from the same subject A as in Example was used.) The changes in the pressure in the pump109 are shown in Fig. 7.

### Examples 4 to 6)

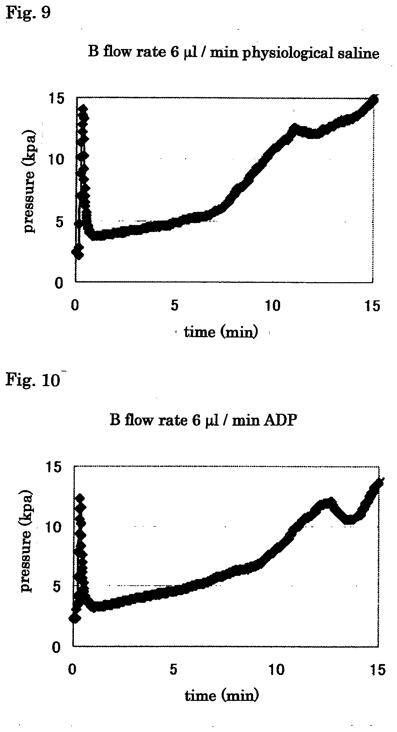

The experiments in Examples 1 to 3 were carried out by measurement using samples from another subject (subject B). The changes in the pressure in the pump109 are shown in Figs. 8, 9 and 10, respectively.

### Comparative Experiment)

Blood from the subjects A and B were centrifuged at 800 rpm to obtain platelet-rich plasma, and ADP-induced platelet aggregability was measured using an aggregometer (PA-20, Kowa Company, Ltd.). Large aggregates induced by 1 µM ADP in the blood from the subject A were about a half of those in the blood from the subject B.

### Discussion)

Based on the results on the subjects A and B, it was confirmed that pressure increase due to adhesion and aggregation to collagen under shear stress was weaker in the subject A than in the subject B. However, in the subject A, aggregability due to ADP was stronger, and pressure increase due to adhesion and aggregation to collagen upon addition of ADP was confirmed to be equivalent to that of the subject B.

### Example 7)

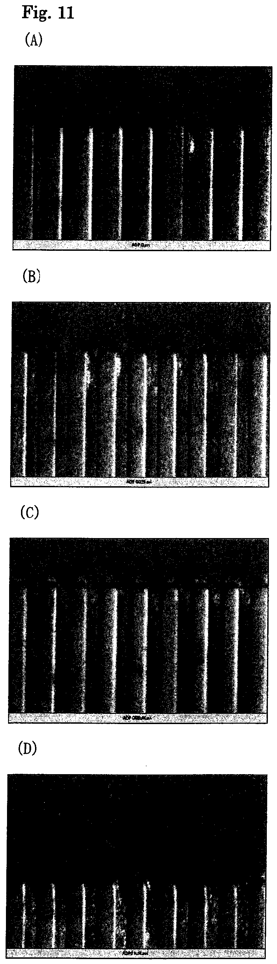

An experiment was carried out using the microchip 1 and the system A in Example 1 for diagnosis by images. The pump 109 was filled with mineral oil, and the reservoir 107 was filled with blood anticoagulated with citric acid. The mineral oil was sent to the reservoir 107 using the pump 109 at a flow rate of 8 µl/min. to allow the blood to flow into the microchip 1. Exactly the same experiments were carried out using blood prepared by adding ADP to citric acid-treated blood to final concentrations of 0.025 µM, 0.05 µM and 0.1 µM, respectively. Each panel in Fig. 11 shows an image of the state inside the microchip 1 after 1 minute of the experiment, which was taken by the camera 111.

### Discussion)

When ADP was added, increase in the amount of platelets adhered to the comb portion, which occurred in an ADP concentration-dependent manner, was observed, and it was possible to visually confirm the adherence of platelets even at levels at which pressure increase did not occur.

### [Preparation of Microchip and Platelet Function Testing Device]

The first substrate 200 shown in Fig. 3A and the second substrate 210 shown in Fig. 3B were laminated with and bonded to each other, to provide the microchip 2 shown in Fig. 3C. In terms of the substrate 200, a substrate made of PDMS (Fluidware) was used, and the depth of all the channels was 120 µm; the channel width was 1 mm; the length of the comb was 1 mm; the width of the comb was 50 µm; and the groove width was 50 µm. In terms of the substrate 210, a slide glass was used, and penetrating holes corresponding to the inlet and the outlet were provided as in the substrate 110, but, unlike the substrate 110, collagen was not applied since the glass plays a role as a platelet-adhesive surface. That is, the total length of the second substrate side in the capillary 201 works as a platelet-adhesive surface 206.

The platelet function testing device B shown in Fig. 4 is the same as the platelet function testing device A in Fig. 2 except that the microchip 2 was used instead of the microchip 1 and that it does not have a stirring bar and a stirrer. Since, in the Examples below, anticoagulated blood preliminarily mixed with ADP was placed in the reservoir, a stirring bar and a stirrer were not necessary.

### Example 8)

The microchip 2 shown in Fig. 3 and the platelet function testing device B shown in Fig. 4 were used. About 600 µl of blood anticoagulated with citric acid was filled in a blood reservoir 207. A pump 209 was filled with mineral oil, and the pump 209 was connected to the reservoir 207, whose tip was further connected to an inlet 204 in the microchip 2. The mineral oil was injected from the pump 209 into the microchip 2 at a flow rate of 20 µl/min., and the inflow pressure of the mineral oil was measured by a pressure sensor which is not shown.

### Example 9)

Pressure changes were measured by carrying out an experiment which was the same as that in Example 8 except that ADP (manufactured by Wako Pure Chemical Industries, Ltd.) was further added to the blood anticoagulated with citric acid in the reservoir 207 to a final concentration of 2 nM.

### Example 10)

Pressure changes were measured by carrying out an experiment which was the same as that in Example 9 except that blood collected 3 hours after dosing with 75 mg clopidogrel (Sanofi-aventis) was used.

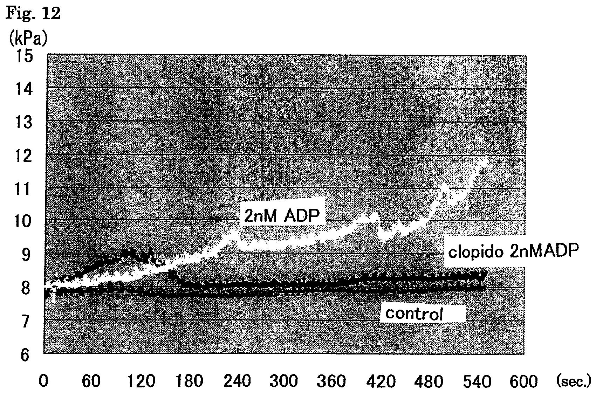

Changes in the pressure in the pump 209 in Examples 8 to 10 are shown in Fig. 12. Addition of a very small amount of ADP caused pressure increase, and the increase was suppressed by administration of clopidogrel.

### [Preparation of Microchip and Platelet Function Testing Device]

Two transparent substrates, that is, the first substrate 100 shown in Fig. 1(A) and the second substrate 110 shown in Fig. 1(B) (injection-molded products manufactured by Richell Corporation) were prepared. The side of the first substrate 100 wherein a groove corresponding to a capillary 101 opens and the side of the second substrate 110 having a platelet-adhesive surface 106 were bonded together such that these face to each other using a silane coupling agent and thermocompression bonding, to provide the microchip 1 shown in Fig. 1(C). In the first substrate 100, the length, depth and width of the channel (capillary) 101 were 20 mm, 50 µm and 2 mm, respectively. In terms of the channel dividing section 102, dividing walls 103 each having a length of 1.5 mm, width of 50 µm and height of 50 µm were placed at equal intervals of 50 µm, and this part was used as the channel dividing section. In the second substrate, each of the holes corresponding to the inlet and the outlet was a penetrating hole having a circular cross section and an inner diameter of 2 mm and a depth of 2 mm. At the position on the second substrate 110 which is overlapped with the channel dividing section 102 of the first substrate 100, about 10 µl of 3 mg/ml collagen type I (manufactured by Nitta Gelatin Inc.) was applied and dried under vacuum to provide a platelet-adhesive surface 106. The area in which collagen was applied was from 4 mm upstream of the comb to 2 mm downstream of the combs, including the comb.

As shown in Fig. 4, the prepared microchip 1 was placed on a heater 215, and a pump209 and a reservoir 207 were connected to the microchip 1 as in Example 8.

### Example 11)

(a) Blood was collected from the subject D using a hirudin blood collection tube (Multiplate Services GmbH), and about 600 µl of the blood anticoagulated with hirudin was filled in the reservoir 207. The pump 209 was filled with mineral oil and connected to the reservoir 207, whose tip was further connected to the inlet 104 in the microchip 1. The mineral oil was injected from the pump 209 into the reservoir at a flow rate of 200 µl/min. for the first 5 seconds, and 20 µl/min. thereafter, thereby injecting the blood in the reservoir into the microchip 1 at the same flow rates, while measuring the inflow pressure of the mineral oil with a pressure sensor which is not shown.
(b) An experiment was carried out in the same manner as in (a) except that mineral oil was injected to the reservoir at a flow rate of 200 µl/min. for the first 5 seconds, and 10 µl/min. thereafter, thereby injecting the blood in the reservoir into the microchip 1 at the same flow rates, while measuring the inflow pressure of the mineral oil.
(c) An experiment was carried out in the same manner as in (a) except that mineral oil was injected to the reservoir at a flow rate of 200 µl/min. for the first 5 seconds, and 5 µl/min. thereafter, thereby injecting the blood in the reservoir into the microchip 1 at the same flow rates, while measuring the inflow pressure of the mineral oil.

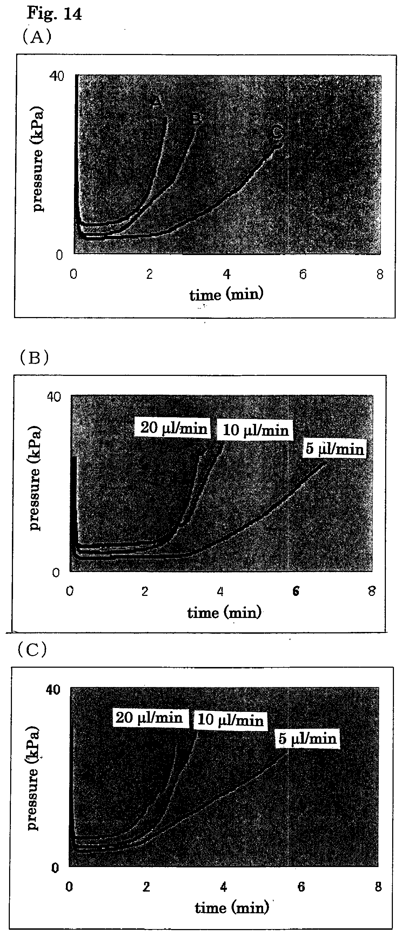

The results of Example 11 (a), (b) and (c) are shown in Fig. 14(A). The abscissa indicates time, and the ordinate indicates pressure increase due to obstruction in the chip. Pressure increase occurred in the subject D in the order of Examples (a), (b) and (c), that is, in descending order of shear stress.

### Example 12)

The same experiment as in Example 11 was carried out also with the subject E. The results are shown in Fig. 14(B).

### Example 13)

The same experiment as in Example 11 was carried out also with the subject F. The results are shown in Fig. 14(C).

### Reference Example)

Platelet aggregability was measured for the subjects D, E and F using a whole-blood aggregometer Multiplate (Dynabyte). Blood was collected using a hirudin blood collection tube, and aggregability induced by 6 µM ADP was measured. The results were as follows: D, 691 AU/min.; E, 525 AU/min.; and F, 652 AU/min. ADP-induced aggregability was strongest in the subject D, followed by the subject F and the subject E in that order.

### Discussion

In any of D, E and F, pressure increase first occurred under high shear stress, followed by medium and low shear stress in that order. Further, the pressure increase first occurred in the subject D, followed by F and E in that order, and this result was correlated with the result of the test on ADP-induced aggregability carried out with Multiplate.

An experiment was carried out in the same manner as in Example 11a except that blood was collected with a citric acid blood collection tube and anticoagulated with sodium citrate. In this case, pressure increase was not observed even 10 minutes later.

### Example 14)

About 600 µl of blood of the subject E anticoagulated with citric acid was mixed with 6 µl of 100 µM ADP reagent (Chrono-log Corporation), and immediately thereafter, the reservoir was filled with the blood. A pump filled with mineral oil was connected to the reservoir, which was further connected to the microchip, as in Example 11a. The microchip 1 was placed on a heater, and the blood was allowed to flow at a flow rate of 200 µl/min. for the first 5 seconds, and 20 µl/min. thereafter.

### Example 15)

a) An experiment was carried out in the same manner as in Example 14 except that blood of the subject D was used.
b) An experiment was carried out in the same manner as in Example 14 except that blood of the subject D collected 6 hours after oral administration of 100 mg of clopidogrel was used.

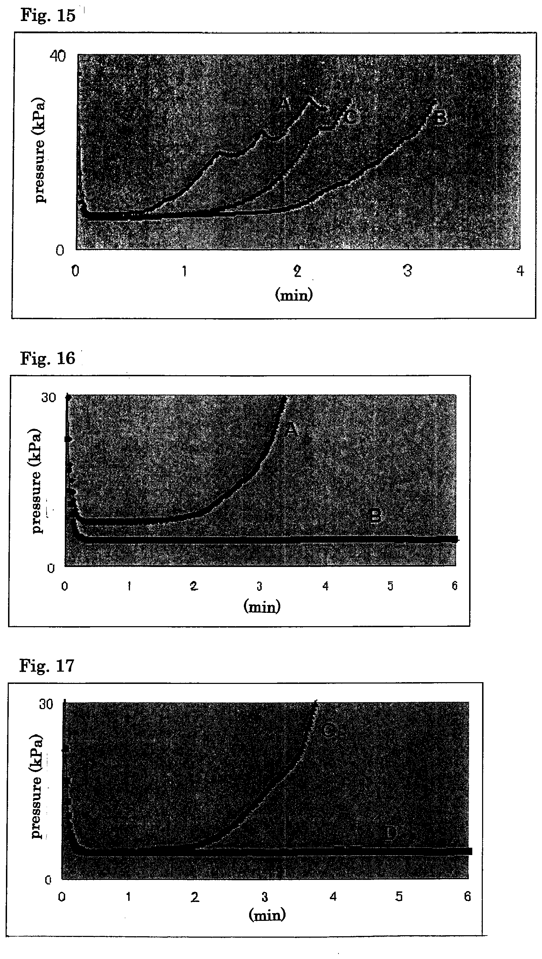

The results of Examples 15 and 14 are shown in Fig. 15. In Fig. 5, A indicates the results of Example 15a); B indicates the results of Example 15b; and C indicates the results of Example 14. It can be seen that, in the subject D, pressure increase delayed due to administration of clopidogrel, and that the pressure increase after the administration of clopidogrel was even slower than that of the blood of the subject E.

### Example 16)

a) About 600 µl of blood of the subject G anticoagulated with citric acid was mixed with 6 µl of 50 mM arachidonic acid (Chrono-log Corporation), and immediately thereafter, the reservoir was filled with the blood. A pump filled with mineral oil was connected to the reservoir, which was further connected to the microchip, as in Example 11a. The microchip 1 was placed on a heater, and the blood was allowed to flow at a flow rate of 200 µl/min. for the first 5 seconds, and 20 µl/min. thereafter.
b) About 600 µl of blood of the subject G anticoagulated with citric acid was mixed with 6 µl of 50 mM arachidonic acid (Chrono-log Corporation), and immediately thereafter, the reservoir was filled with the blood. A pump filled with mineral oil was connected to the reservoir, which was further connected to the microchip, as in Example 11a. The microchip 1 was placed on a heater, and the blood was allowed to flow at a flow rate of 200 µl/min. for the first 5 seconds, and 10 µl/min. thereafter.
c) About 600 µl of blood of the subject G anticoagulated with citric acid was mixed with 6 µl of 50 mM arachidonic acid (Chrono-log Corporation), and immediately thereafter, the reservoir was filled with the blood. A pump filled with mineral oil was connected to the reservoir, which was further connected to the microchip, as in Example 11a. The microchip 1 was placed on a heater, and the blood was allowed to flow at a flow rate of 200 µl/min. for the first 5 seconds, and 5 µl/min. thereafter.
d) About 600 µl of blood of the subject G anticoagulated with citric acid was mixed with 6 µl of 100 µg/ml collagen (Chrono-log Corporation), and immediately thereafter, the reservoir was filled with the blood. A pump filled with mineral oil was connected to the reservoir, which was further connected to the microchip, as in Example 11a. The microchip 1 was placed on a heater, and the blood was allowed to flow at a flow rate of 200 µl/min. for the first 5 seconds, and 20 µl/min. thereafter.
e) About 600 µl of blood of the subject G anticoagulated with citric acid was mixed with 6 µl of 100 µg/ml collagen (Chrono-log Corporation), and immediately thereafter, the reservoir was filled with the blood. A pump filled with mineral oil was connected to the reservoir, which was further connected to the microchip, as in Example 11a. The microchip 1 was placed on a heater, and the blood was allowed to flow at a flow rate of 200 µl/min. for the first 5 seconds, and 10 µl/min. thereafter.
f) About 600 µl of blood of the subject G anticoagulated with citric acid was mixed with 6 µl of 100 µg/ml collagen (Chrono-log Corporation), and immediately thereafter, the reservoir was filled with the blood. A pump filled with mineral oil was connected to the reservoir, which was further connected to the microchip, as in Example 11a. The microchip 1 was placed on a heater, and the blood was allowed to flow at a flow rate of 200 µl/min. for the first 5 seconds, and 5 µl/min. thereafter.

### Example 17)

The experiments a to f in Example 16 were carried out for the subject G 6 hours after dosing with 100 mg of aspirin.

### Example 18)

Using blood of the subject G collected 6 hours after dosing with 100 mg of aspirin, experiments were carried out in the same manner as in Examples 16d to f except that the collagen to be added was increased to 12 µl or 18 µl.

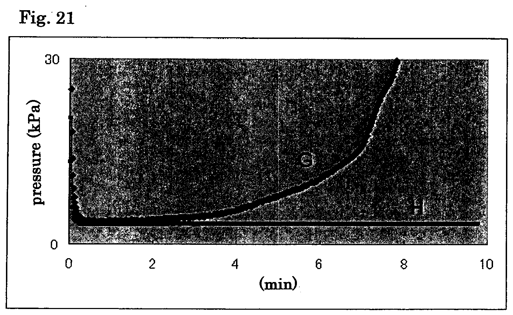

The results of Example 16 to 18 are shown in Figs. 16 to 21.

From these results, it was shown that the anti-platelet effect of aspirin can be analyzed by adding arachidonic acid or collagen to citric acid-treated blood and allowing the resulting mixture to flow in the microchip, while measuring pressure increase. Further, as shown in Fig. 19, it was revealed that blood collected after dosing with aspirin can also be made to cause pressure increase by increasing the collagen to be added. Therefore, it was revealed that the method of the present invention allows evaluation of not only the presence/absence of the effect of aspirin but also the extent of suppression of platelet function.

Thus, by combinational diagnosis based on visual confirmation of activated-platelet aggregates by imaging ability and on pressure increase, it is possible to grasp the condition of patient platelets in more detail.

### Example 19)

An experiment was carried out in the same manner as in Example 11(a) except that blood of the subject H was used.

### Example 20)

An experiment was carried out in the same manner as in Example 19 except that mineral oil was injected from the pump 209 into the reservoir at a flow rate of 200 µl/min. for the first 5 seconds, and 7 µl/min. thereafter, thereby injecting the blood in the reservoir into the microchip 1 at the same flow rates, while measuring the inflow pressure of the mineral oil.

### Example 21)

Measurement was carried out in the same manner as in Example 19 and Example 20 except that 0.4 µg/ml and 0.8 µg/ml, respectively, ReoPro (Eli lilly), which is an antibody against the receptor GPIIb/IIIa on the platelet membrane, was added to blood anticoagulated with hirudin.

### Example 22)

Measurement was carried out in the same manner as in Example 19 and Example 20 except that 0.01 µg/ml and 0.1 µg/ml, respectively, OS-1 (Biochemistry. 2008 Apr 22;47(16):4674-82), which is an inhibitor of the receptor GPIb on the platelet membrane, was added to blood anticoagulated with hirudin.

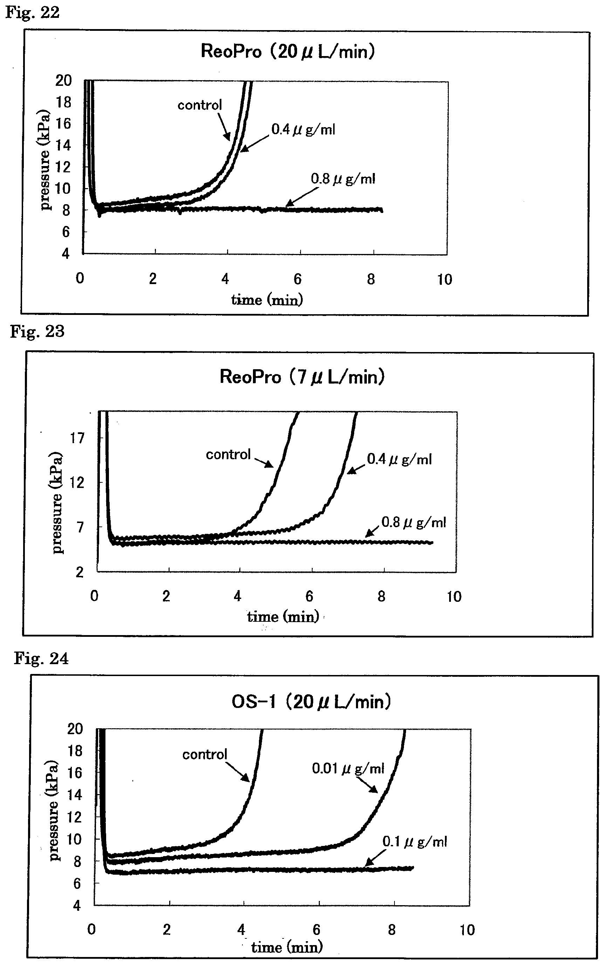

The results of Examples 19, 20 and 21 are shown in Fig. 22 and Fig. 23.

Fig. 22 shows the inhibitory effect of ReoPro at a flow rate of 20 µl/min., and Fig. 23 shows the inhibitory effect of ReoPro at a flow rate of 7 µl/min.

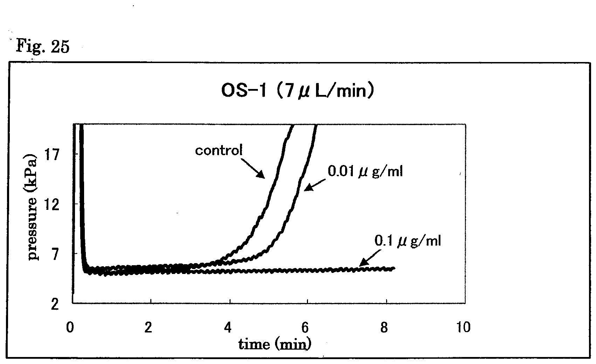

The results of Example 22 are shown in Fig. 24 and Fig. 25.

Fig. 24 shows the inhibitory effect of OS-1 at a flow rate of 20 µl/min., and Fig. 25 shows the inhibitory effect of OS-1 at a flow rate of 7 µl/min.

### Discussion)

GPIIb/IIIa binds mainly to fibrinogen and is involved in platelet aggregation, and GPIb is a receptor which binds to vWF and is involved in the platelet adhesion reaction under high shear stress.

OS-1, which suppresses the platelet adhesion reaction, showed a strong anti-platelet action under high shear stress, while ReoPro showed a rather strong anti-platelet effect under low shear stress.

### DESCRIPTION OF SYMBOLS

A, B: platelet monitoring device; 1, 2, 3: microchip; 100, 200, 300: first substrate; 101, 201, 301: capillary; 102, 202, 302: channel dividing section; 103, 203, 303: channel dividing walls; 104, 204, 304: inlet; 105, 205: outlet; 106, 206, 306: platelet-adhesive surface; 107, 207: reservoir; 108: stirring bar; 109, 209: pump; 110, 210: second substrate; 111, 211: camera; 112, 212: outlet duct; 113: stirrer; 114, 214: image analyzer; 115, 215: heater; 305: air hole; 307: waste liquid reserving section; 308: blood absorbing material

## Claims

1. A method for testing platelet aggregation, comprising subjecting anticoagulated blood to a weak platelet-activation treatment with a platelet-activating reagent, passing the treated anticoagulated blood through a capillary having a platelet-adhesive surface on at least a part of its inner surface, and observing or measuring the behavior of the blood in the capillary to test platelet aggregation,
wherein at least a part of the inside of said capillary has a section comprising walls which extend along the direction of the blood flow in the capillary and divide the width of the capillary into not less than 5 channels, wherein the width of each channel in said section is 10 to 200 µm,
wherein said platelet-adhesive surface is provided in said section of the capillary, and
wherein said weak platelet-activation treatment is carried out by mixing the anticoagulated blood with a platelet-activating reagent in an amount with which irreversible platelet aggregation does not occur.

2. The method according to claim 1, wherein said anticoagulated blood is a whole blood treated with an anticoagulant selected from citric acid, heparin or hirudin.

3. The method according to claim 1 or 2, wherein said platelet-adhesive surface is made of collagen coating or made of glass.

4. The method according to claim 1, wherein said platelet-activating reagent is adenosine diphosphate, which is mixed with the anticoagulated blood to a concentration of 0.001 to 5 µM or said platelet-activating reagent is arachidonic acid, which is mixed with the anticoagulated blood to a concentration of 0.001 to 1 mM.

5. The method according to any of claims 1 to 4, wherein said capillary is formed in a microchip.

6. The method according to any of claims 1 to 5, wherein said anticoagulated blood is introduced into the capillary by a pump, and the pressure exerted on the pump by inflow of the blood into the capillary is measured with a pressure sensor, thereby testing platelet aggregation.

7. The method according to claim 6, wherein said anticoagulated blood is stored in a blood storage section connected to the capillary and the pump, which blood is introduced into the capillary by introduction of a liquid having a specific gravity smaller than the blood into the blood storage section by the pump, and the inflow pressure of the liquid is measured, thereby indirectly measuring the pressure exerted by inflow of the blood into the capillary.

8. The method according to claim 6, wherein said anticoagulated blood is stored in a blood storage section connected to the capillary and the pump, which anticoagulated blood is mixed with the platelet-activating reagent in the blood storage section by introduction of the platelet-activating reagent by said pump into the blood storage section, and the blood mixed with the platelet-activating reagent is introduced into the capillary, while measuring the inflow pressure of the platelet-activating reagent, thereby indirectly measuring the pressure exerted by inflow of the blood into the capillary.

9. The method according to claim 8, wherein said platelet-activating reagent is mixed with the anticoagulated blood such that the concentration of the platelet-activating reagent in the mixture of the anticoagulated blood and the platelet-activating reagent increases along a linear concentration gradient or a stepwise concentration gradient.

10. The method according to claim 8 or 9, wherein the mixture of the anticoagulated blood and the platelet-activating reagent is stirred.

## Patentansprüche

1. Verfahren zum Testen einer Blutplättchenaggregation, mit den Schritten zum Unterziehen von antikoaguliertem Blut einer schwachen Blutplättchenaktivierungsbehandlung mit einem Blutplättchenaktivierungsreagens, Leiten des behandelten antikoagulierten Blutes durch eine Kapillare mit einer Blutplättchenanhaftungsfläche auf mindestens einem Teil ihrer Innenfläche und Beobachten oder Messen des Verhaltens des Blutes in der Kapillare zum Testen der Blutplättchenaggregation,
wobei mindestens ein Teil des Innenraums der Kapillare einen Abschnitt aufweist, der Wände aufweist, die sich entlang der Richtung des Blutflusses in der Kapillare erstrecken und die Breite der Kapillare in nicht weniger als 5 Kanäle teilen, wobei die Breite jedes Kanals in dem Abschnitt 10 bis 200 µm beträgt,
wobei die Blutplättchenanhaftungsfläche in dem Abschnitt der Kapillare vorgesehen ist, und
wobei die schwache Blutplättchenaktivierungsbehandlung durch Mischen des antikoagulierten Blutes mit einem Blutplättchenaktivierungsreagens in einer Menge ausgeführt wird, bei der keine irreversible Blutplättchenaggregation auftritt.

2. Verfahren nach Anspruch 1, wobei das antikoagulierte Blut Vollblut ist, das mit einem Antikoagulans behandelt ist, das aus Zitronensäure, Heparin oder Himdin ausgewählt ist.

3. Verfahren nach Anspruch 1 oder 2, wobei die Blutplättchenanhaftungsfläche aus einer Kollagenbeschichtung oder aus Glas besteht.

4. Verfahren nach Anspruch 1, wobei das Blutplättchenaktivierungsreagens Adenosindiphosphat ist, das mit dem antikoagulierten Blut in einer Konzentration von 0,001 bis 5 µM gemischt ist, oder wobei das Blutplättchenaktivierungsreagens Arachidonsäure ist, die mit dem antikoagulierten Blut in einer Konzentration von 0,001 bis 1 mM gemischt ist.

5. Verfahren nach einem der Ansprüche 1 bis 4, wobei die Kapillare in einem Mikrochip ausgebildet ist.

6. Verfahren nach einem der Ansprüche 1 bis 5, wobei das antikoagulierte Blut durch eine Pumpe in die Kapillare eingeleitet wird und der Druck, der durch Einströmen des Bluts in die Kapillare auf die Pumpe ausgeübt wird, durch einen Drucksensor gemessen wird, um dadurch die Blutplättchenaggregation zu testen.

7. Verfahren nach Anspruch 6, wobei das antikoagulierte Blut in einem mit der Kapillare und der Pumpe verbundenen Blutspeicherabschnitt gespeichert ist, wobei das Blut in die Kapillare eingeleitet wird, indem eine Flüssigkeit mit einem spezifischen Gewicht, das kleiner ist als dasjenige des Bluts, durch die Pumpe in den Blutspeicherabschnitt eingeleitet wird, wobei der Einströmdruck der Flüssigkeit gemessen wird, wodurch indirekt der Druck gemessen wird, der durch Einströmen des Bluts in die Kapillare ausgeübt wird.

8. Verfahren nach Anspruch 6, wobei das antikoagulierte Blut in einem mit der Kapillare und der Pumpe verbundenen Blutspeicherabschnitt gespeichert ist, wobei das antikoagulierte Blut mit dem Blutplättchenaktivierungsreagens in dem Blutspeicherabschnitt durch Einleiten des Blutplättchenaktivierungsreagens durch die Pumpe in den Blutspeicherabschnitt gemischt wird, und wobei das mit dem Blutplättchenaktivierungsreagens gemischte Blut in die Kapillare eingeleitet wird, während der Einströmdruck des Blutplättchenaktivierungsreagenz gemessen wird, wodurch indirekt der Druck gemessen wird, der durch Einströmen des Bluts in die Kapillare ausgeübt wird.

9. Verfahren nach Anspruch 8, wobei das Blutplättchenaktivierungsreagens mit dem antikoagulierten Blut gemischt wird, so dass die Konzentration des Blutplättchenaktivierungsreagens im Gemisch aus dem antikoagulierten Blut und dem Blutplättchenaktivierungsreagens entlang eines linearen Konzentrationsgradienten oder eines schrittweisen Konzentrationsgradienten zunimmt.

10. Verfahren nach Anspruch 8 oder 9, wobei das Gemisch aus dem antikoagulierten Blut und dem Blutplättchenaktivierungsreagenz gerührt wird.

## Revendications

1. Procédé pour tester l'agrégation des plaquettes, comprenant l'exposition d'un sang traité par anticoagulation à un traitement d'activation des plaquettes faible avec un réactif activant les plaquettes, le passage du sang traité par anticoagulation traité à travers un capillaire ayant une surface adhésive pour les plaquettes sur au moins une partie de sa surface interne, et l'observation ou la mesure du comportement du sang dans le capillaire pour tester l'agrégation des plaquettes,
où au moins une partie de l'intérieur dudit capillaire a une section comprenant des parois qui s'étendent dans la direction de l'écoulement du sang dans le capillaire et divisent la largeur du capillaire en pas moins de 5 canaux, où la largeur de chaque canal dans ladite section est 10 à 200 µm,
où ladite surface adhésive pour les plaquettes est prévue dans ladite section du capillaire, et
où ledit traitement d'activation des plaquettes faible est réalisé par mélange du sang traité par anticoagulation avec un réactif activant les plaquettes en une quantité avec laquelle une agrégation des plaquettes irréversible ne se produit pas.

2. Procédé selon la revendication 1, où ledit sang traité par anticoagulation est un sang total traité avec un anticoagulant choisi parmi l'acide citrique, l'héparine et l'hirudine.

3. Procédé selon la revendication 1 ou 2, où ladite surface adhésive pour les plaquettes est faite d'un revêtement de collagène ou faite de verre.

4. Procédé selon la revendication 1, où ledit réactif activant les plaquettes est l'adénosine diphosphate, qui est mélangé avec le sang traité par anticoagulation jusqu'à une concentration de 0,001 à 5 µM ou ledit réactif activant les plaquettes est l'acide arachidonique, qui est mélangé avec le sang traité par anticoagulation jusqu'à une concentration de 0,001 à 1 mM.

5. Procédé selon l'une quelconque des revendications 1 à 4, où ledit capillaire est formé dans une micropuce.

6. Procédé selon l'une quelconque des revendications 1 à 5, où ledit sang traité par anticoagulation est introduit dans le capillaire par une pompe, et la pression exercée sur la pompe par l'entrée du sang dans le capillaire est mesurée avec un capteur de pression, testant ainsi l'agrégation des plaquettes.

7. Procédé selon la revendication 6, où ledit sang traité par anticoagulation est stocké dans une section de stockage de sang reliée au capillaire et à la pompe, lequel sang est introduit dans le capillaire par introduction d'un liquide ayant une masse volumique inférieure au sang dans la section de stockage de sang par la pompe, et la pression d'entrée du liquide est mesurée, mesurant ainsi indirectement la pression exercée par l'entrée du sang dans le capillaire.

8. Procédé selon la revendication 6, où ledit sang traité par anticoagulation est stocké dans une section de stockage de sang reliée au capillaire et à la pompe, lequel sang traité par anticoagulation est mélangé avec le réactif activant les plaquettes dans la section de stockage de sang par introduction du réactif activant les plaquettes par ladite pompe dans la section de stockage de sang, et le sang mélangé avec le réactif activant les plaquettes est introduit dans le capillaire, tout en mesurant la pression d'entrée du réactif activant les plaquettes, mesurant ainsi indirectement la pression exercée par l'entrée du sang dans le capillaire.

9. Procédé selon la revendication 8, où ledit réactif activant les plaquettes est mélangé avec le sang traité par anticoagulation de sorte que la concentration du réactif activant les plaquettes dans le mélange du sang traité par anticoagulation et du réactif activant les plaquettes augmente suivant un gradient de concentration linéaire ou un gradient de concentration par pas.

10. Procédé selon la revendication 8 ou 9, où le mélange du sang traité par anticoagulation et du réactif activant les plaquettes est agité.
